# EUROPÄISCHE PATENTSCHRIFT

(11) **EP 2 521 115 B1**
(45) Veröffentlichungstag und Bekanntmachung des Hinweises auf die Patenterteilung: **13.11.2019**
(21) Anmeldenummer: 12165541.9
(22) Anmeldetag: 25.04.2012
(51) Int. Cl.: G09F 3/10, A61M 5/178, G09F 3/02

(54) **Kennzeichnungsetikett für ein zylinderförmiges Gefäß und zylinderförmiges Gefäß**
Identification label for a cylindrical container and cylindrical container
Étiquette de marquage pour un récipient cylindrique et récipient cylindrique

(30) Priorität: 02.05.2011 DE 202011100113 U
(43) Veröffentlichungstag der Anmeldung: 07.11.2012
(73) Patentinhaber: Schreiner Group GmbH & Co. KG, 85764 Oberschleissheim (DE)
(72) Erfinder: Seidl, Peter, 81371 München (DE)
(74) Vertreter: Epping - Hermann - Fischer

(56) Entgegenhaltungen:
- EP-A1- 1 044 698
- WO-A1-2009/137756
- US-A1- 2003 134 073
- US-A1- 2006 019 052

## Beschreibung

Die vorliegende Erfindung betrifft ein Kennzeichnungsetikett für ein zylinderförmiges Gefäß, sowie ein zylinderförmiges Gefäß das mit einem Kennzeichnungsetikett versehen ist. Insbesondere betrifft die Erfindung ein Kennzeichnungsetikett für vorzugsweise dünne Injektionsspritzen und Injektionsspritzen, die mit einem solchen Etikett gekennzeichnet sind.

Das Dokument WO 2009/137756 A1 offenbart ein selbst-laminierendes Etikett zum Kennzeichnen eines Kabels, welches auf einfache Weise um das Kabel gewickelt werden kann. Ein Teil des Etiketts ist im angebrachten Zustand drehbar um das Kabel angeordnet, sodass eine Beschriftung an radial verschiedenen Positionen ausgelesen werden kann. Der zweite und dritte Abschnitt bilden in einem applizierten Zustand eine drehbare Hülse um das Kabel aus. In der Druckschrift US 2006/019052 A1 ist ein Etikett zum Kennzeichnen von Schmuck beschrieben, welches einen länglichen Abschnitt aufweist, der bei einem Anbringen des Etiketts gebogen wird und mittels eines zweiten Abschnitts 16 zwischen einem ersten und einem Abschnitt klebend versiegelt wird.

Im pharmazeutischen und medizinischen Bereich kommt der Kennzeichnung einzelner Produkte, wie beispielsweise Medikamenten, Blutproben oder ähnlichem eine sehr hohe Bedeutung zu. Da diese Produkte in der Regel eine ganz bestimmte Person betreffen und Verwechslungen mit sehr großen Risiken verbunden sind, muss von Anfang an sichergestellt sein, dass jedes Produkt zu jedem Zeitpunkt klar gekennzeichnet ist. Somit soll insbesondere auch unter Stresssituationen eine mögliche Verwechslung und zum Teil lebensgefährliche Risiken vermieden werden.

Für die Sicherheit der gekennzeichnet pharmazeutischen und medizinischen Produkte ist es darüber hinaus auch von großer Bedeutung, dass die Kennzeichnung während der gesamten Lebensdauer sicher mit dem Produkt verbunden ist und sich nicht beispielsweise ein aufgeklebtes Etikett einfach ablöst.

Außerdem soll die Kennzeichnung stets gut lesbar sein. Die Kennzeichnung sollte nicht durch irgendwelche Umwelteinflüsse oder ähnliches so in Mitleidenschaft gezogen werden, dass die Beschriftung unleserlich würde oder es zu Verwechslungen kommen könnte.

Darüber hinaus sollte eine moderne Kennzeichnung auch eine schnelle und effiziente Bearbeitung ermöglichen. Das heißt, dass die Kennzeichnung maschinell erstellt werden kann. Hierzu eignen sich unterschiedliche Verfahren einer automatisierten Bedruckung. Beispielsweise kann ein entsprechendes Etikett bereits während der Herstellung gleichzeitig auch mit einer Kennzeichnung bedruckt werden. Aber auch eine ganz individuelle separate Beschriftung, beispielsweise mittels eines Tintenstrahl- oder Thermotransferdrucks, ist möglich. Insbesondere bei einer nachträglichen Beschriftung mittels Tintenstrahldrucker kann die Beschriftung durch Umwelteinflüsse, wie beispielsweise Feuchtigkeit, sehr leicht beschädigt werden. Daher ist in diesen Fällen ein geeigneter Schutz erforderlich.

Moderne Produktions- und Verarbeitungstechniken erfordern auch sehr häufig eine automatisierte Erkennung von bereits gekennzeichneten Produkten. Hierzu werden neben der optischen Zeichenerkennung (OCR) sehr häufig auch Lesegeräte für Barcodes oder Datamatrixcodes verwendet. Für diese optische Erkennungsmethoden ist es jedoch erforderlich, dass der Kennzeichnungsbereich eines Produkts möglichst plan vor das Erkennungsgerät geführt wird. Insbesondere bei zylinderförmigen Gefäßen mit einem engen Radius, wie beispielsweise kleinen, dünnen Injektionsspritzen kann dies in der Regel nicht gewährleistet werden. Die Kennzeichnung erstreckt sich in diesen Fällen meist über mehr als den halben Umfang, so dass eine automatisierte Erkennung nicht oder nur mit erheblichem Aufwand möglich ist.

Es ist daher eine Aufgabe der vorliegenden Erfindung, ein Kennzeichnungsetikett bereitzustellen, das auch für zylinderförmige Gefäße mit einem engen Radius eine zuverlässige automatisierte Erkennung ermöglicht und dabei das Kennzeichnungsetikett dennoch dauerhaft sicher mit dem Gefäß verbunden bleibt.

Dies wird durch ein Kennzeichnungsetikett gemäß Anspruch 1 gelöst. Dieses Etikett umfasst einen ersten, einen zweiten und einen dritten Etikettenabschnitt. Ferner sind eine oder mehrere Stanzlinien vorgesehen, durch die der zweite Etikettenabschnitt von dem ersten Etikettenabschnitt und von dem dritten Etikettenabschnitt getrennt ist. Der erste Etikettenabschnitt umfasst eine zumindest teilweise mit Klebstoff beschichtete Unterseite. Der zweite Etikettenabschnitt ist zumindest teilweise opak und umfasst eine zumindest teilweise nicht klebende Unterseite. Der dritte Etikettenabschnitt ist zumindest teilweise transparent und umfasst eine zumindest teilweise mit Klebstoff beschichtete Unterseite.

Der Übergang von einer ersten Außenkante des ersten Etikettenabschnitts zu einer zweiten Außenkante des dritten Etikettenabschnitts weist einen abgerundeten Kantenverlauf auf. Erfindungsgemäß ist ferner vorgesehen, dass der zweite Etikettenabschnitt nur durch einen nicht eingestanzten Verbindungsbereich mit dem ersten Etikettenabschnitt verbunden ist und dass in einem auf einem zylinderförmigen Gefäß applizierten Zustand des Kennzeichnungsetiketts der dritte Etikettenabschnitt über dem zweiten Etikettenabschnitt zu liegen kommt. Dadurch ist mittels des zweiten und dritten Etikettenabschnitts eine Fahne ausbildbar, welche im applizierten Zustand des Etiketts von dem zylinderförmigen Gefäß absteht.

Es ist ein Ansatz dieser Erfindung, dass das Kennzeichnungsetikett einerseits aufgrund der nicht klebenden Unterseite des zweiten Etikettenabschnitts einen Etikettenteil besitzt, der mit dem zu kennzeichnenden Produkt nicht vollständig verbunden ist, sondern von dem Zylinder des Produkts absteht. Somit kann sich eine annähernd ebene, gerade Fahne ausbilden. Diese Fahne kann von einem Menschen oder einer Maschine wesentlich leichter erfasst und gelesen werden als ein vollständig auf der gekrümmten Mantelfläche aufgeklebtes Etikett.

Andererseits wird an den besonders einreißgefährdeten Stellen des Etiketts auf scharfkantige Winkel verzichtet und diese Ecken durch einen abgerundeten Linienverlauf der äußeren Stanzlinien ersetzt. Hierdurch wird das Risiko eines Einreißens der Etikettenfolie deutlich minimiert. Insbesondere sinkt die Gefahr, dass der Teil des Kennzeichnungsetiketts, der von dem etikettierten Produkt absteht, leicht abgetrennt wird und somit die Kennzeichnung verloren geht.

Vorzugsweise ist die Unterseite des zweiten Etikettenabschnitts mit Klebstoff beschichtet und die Wirkung des Klebstoffs ist hier zumindest teilweise neutralisiert. Somit kann für die Herstellung des Kennzeichnungsetiketts das gesamte Etikett zunächst vollflächig mit Klebstoff beschichtet werden. Anschließend wird nur der Teilbereich, an dem keine Klebewirkung gewünscht wird, durch Aufbringungen eines Klebstoffkillers neutralisiert.

Bevorzugt ist die Oberseite des zweiten Etikettenabschnitts zumindest teilweise mit einer opaken Farbe beschichtet ist. Hierdurch wird es ermöglicht, das die aufgebrachten Informationen für die Kennzeichnung deutlich lesbar und mit gutem Kontrast sichtbar sind.

Der erste, der zweite und der dritte Etikettenabschnitt sind vorzugsweise aus einer gemeinsamen Folie oder einem gemeinsamen Folienverbund hergestellt. Vorteilhaft ist hierfür eine Folie aus einem transparenten Kunststoff, beispielsweise Polyester. Die einzelnen Etikettenabschnitte werden durch geeignete Stanzungen in einem einfachen Produktionsverfahren hergestellt.

Vorzugsweise umfasst der zweite Etikettenabschnitt einen Bereich mit aufgedruckten Informationen. Weiterhin bevorzugt sind die Informationen in Form eines maschinenlesbaren Codes aufgedruckt. Somit kann die Kennzeichnung eines Produktes auch in einem automatischen Leseprozess einfach erfasst werden.

In einer bevorzugten Ausführungsform ist ein erfindungsgemäßes Kennzeichnungsetikett auf ein zylinderförmiges Gefäß so aufgebracht, dass die erste Außenkante des ersten Etikettenabschnitts parallel zur Zylinderachse des Gefäßes verläuft. Auf diese Weise erhält man eine zuverlässige und gut lesbare Kennzeichnung eines solchen Gefäßes.

Vorzugsweise ist das Kennzeichnungsetikett so auf dem Gefäß aufgebracht, dass der dritte Etikettenabschnitt den zweiten Etikettenabschnitt zumindest teilweise überdeckt. Somit ist die Kennzeichnung auf dem zweiten Etikettenabschnitt durch den darüber liegenden dritten Abschnitt vor Beschädigungen und Verunreinigungen geschützt.

Der zweite Etikettenabschnitt und der dritte Etikettenabschnitt bilden eine Fahne aus, die in einem applizierten Zustand, wenn das Kennzeichnungsetikett auf dem zylinderförmigen Gefäß aufgebracht ist, von dem zylinderförmigen Gefäß absteht. Aufgrund dieses Abstehens kann die Kennzeichnung besonders gut automatisch erfasst werden.

In einer bevorzugten Ausführungsform ist das zylinderförmige Gefäß ein Spritzenkörper einer Injektionsspritze. Gerade bei dünnen Spritzen sind die Probleme einer zuverlässigen Kennzeichnung für eine automatische Erfassung besonders groß.

Nachfolgend wird die Erfindung anhand der beigefügten schematischen Zeichnungen näher erläutert. Dabei zeigen:
- Figur 1: eine schematische Darstellung einer Draufsicht auf ein erfindungsgemäßes Kennzeichnungsetikett;
- Figur 2: eine schematische Darstellung eines erfindungsgemäßen Etiketts in einer Schrägansicht;
- Figur 3: eine schematische Darstellung eines etikettierten zylinderförmigen Gefäßes in einer Querschnittsansicht; und
- Figur 4: eine schematische Darstellung einer Draufsicht auf eine alternative ausführungsform eines erfindungsgemäßen Kennzeichnungsetiketts.

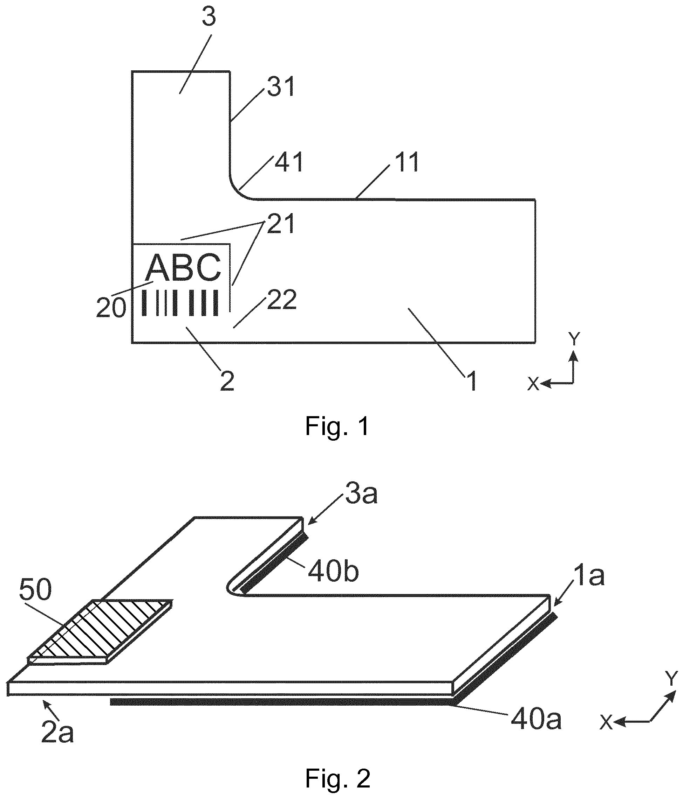

Figur 1 zeigt eine Draufsicht auf ein erfindungsgemäßes Kennzeichnungsetikett. Das Etikett umfasst einen ersten Abschnitt 1, der im Wesentlichen dazu dient, das Etikett an einem zu kennzeichnenden Produkt zu befestigen. Der erste Abschnitt 1 hat üblicherweise eine annähernd rechteckige Form, wobei jeweils zwei Außenkanten parallel zu den Achsen X und Y in Figur 1 verlaufen.

Die Ausdehnung in Richtung Y ist dabei meist etwas größer als der Umfang des zu kennzeichnenden Zylinders. Dies ist jedoch keine zwingende Voraussetzung für die Funktion des Kennzeichnungsetiketts. Auch eine Ausdehnung des ersten Abschnitts 1 in Y-Richtung von weniger als dem Umfang des zu etikettierenden Gefäßes ist möglich, solange eine dabei eine ausreichende Befestigung des Etiketts an dem Gefäß gewährleistet ist.

Weiterhin umfasst das Etikett einen zweiten Abschnitt 2, auf den eine Kennzeichnung 20 des Produkts aufgebracht ist. Der zweite Abschnitt 2 schließt sich in X-Richtung an den ersten Abschnitt 1 an und ist durch die Stanzlinien 21 von dem ersten Anschnitt und dem nachfolgend beschriebenen dritten Abschnitt 3 getrennt und nur an dem nicht eingestanzten Bereich 22 mit dem ersten Etikettenabschnitt 1 verbunden.

Ferner umfasst das Etikett einen transparenten dritten Abschnitt 3, der sich in Y-Richtung an den zweiten Abschnitt 2 anschließt. Der dritte Abschnitt 3 kommt nach Applikation des Kennzeichnungsetiketts über dem zweiten Etikettenabschnitt 2 zu liegen und schützt somit die aufgebrachte Kennzeichnung 20 des zweiten Etikettenabschnitts 2 vor Beschädigungen oder Verschmutzungen.

Vorzugsweise ist das gesamte Kennzeichnungsetikett aus einer vollflächigen transparenten Kunststofffolie hergestellt. Um eine teilweise Opazität des Etiketts zu erhalten, kann das Kennzeichnungsetikett beziehungsweise die Kunststofffolie teilweise mit einer deckenden, opaken Farbe 50 beschichtet werden. Dies ist in Figur 2 für den Bereich des zweiten Etikettenabschnitts 2 dargestellt. Hierzu eignen sich besonders Farben, die mit der später aufzubringenden Kennzeichnung 20 einen hohen Kontrast bildet. Wird die Kennzeichnung 20 beispielsweise in einer dunklen Farbe aufgebracht, so ist für die deckende Farbe 50 eine helle, vorzugsweise weiße Farbe vorteilhaft.

Gleichzeitig kann die deckende Farbe 50 auch als Grundierung dienen, die eine nachträgliche Beschriftung des entsprechenden Bereiches begünstigt oder erst ermöglicht. Insbesondere für die Bedruckung mittels Thermotransfer- oder Tintenstrahldrucks ist eine solche Grundierung von großem Vorteil. Alternativ ist es jedoch auch möglich auf die Farbe 50 eine weitere Grundierungsschicht in einem separaten Produktionsschritt aufzubringen.

Das Aufbringen der deckenden Farbe 50 und gegebenenfalls auch einen zusätzlichen Grundierungsschicht kann hierbei beispielsweise in einem Druckverfahren erfolgen. Somit können die gewünschten Bereiche sehr präzise beschichtet werden.

Die Befestigung des Kennzeichnungsetiketts an einer Injektionsspritze oder einem anderen zylinderförmigen Gefäß erfolgt mittels des ersten Etikettenabschnitts 1. Hinzu befindet sich auf der Unterseite 1a dieses Etikettenabschnitts ein Klebstoff 40a. Somit kann das Kennzeichnungsetikett auf einfache Weise auf die zylinderförmige Außenfläche des zu kennzeichnenden Produkts aufgeklebt werden.

Abhängig von dem speziellen Anwendungsfall kann es vorteilhaft sein, dass der erste Etikettenabschnitt 1 ganz oder zumindest in Teilbereichen transparent ausgeführt ist. Somit verdeckt dieser Teil nicht das etikettierte Gefäß. Ein Benutzer kann auch weiterhin durch das Etikett hindurchsehen und somit den Inhalt des Gefäßes visuell überprüfen. Handelt es sich bei dem Gefäß zum Beispiel um eine Injektionsspritze, so bleibt bei einem transparenten ersten Etikettenabschnitt 1 auch weiterhin die Skala der Injektionsspritze sichtbar.

Alternativ kann der erste Etikettenabschnitt 1 auch opak ausgeführt werden. Somit kann auch dieser Teilbereich mit Informationen bedruckt werden, die auf einem opaken, beispielsweise weißen, Untergrund gut wahrgenommen werden können.

Zum Aufkleben des Etiketts wird das Etikett vorzugsweise mit einer Außenkante parallel zur Zylinderachse des zylinderförmigen Gefäßes aufgelegt. Anschließend wird das Kennzeichnungsetikett entlang der Mantelfläche um das Gefäß herumgewickelt.

Nachdem das Etikett bei diesem Vorgang den Umfang der Mantelfläche einmal vollständig umrundet hat, kommt der dritte Etikettenabschnitt 3 oberhalb des zweiten Etikettenabschnitts 2 zu liegen. Die Unterseite 3a des dritten Etikettenabschnitts 3 ist ebenfalls mit einem Klebstoff 40b versehen, so dass eine feste Verbindung zwischen dem zweiten Etikettenabschnitt 2 und dem dritten Etikettenabschnitt 3 entsteht.

Die Klebstoffbeschichtung 40b des dritten Etikettenabschnitts 3 kann dabei auf gleiche Art erfolgen wie bereits beim ersten Etikettenabschnitt beschrieben.

Da der dritte Etikettenabschnitt 3 transparent ist, bleiben die Kennzeichnungen 20 des zweiten Etikettenabschnitts 2 trotz des Schutzes durch den dritten Etikettenabschnitt 3 sichtbar. Der dritte Etikettenabschnitt 3 dient somit als Schutzlaminat.

Der zweite Etikettenabschnitt 2 ist auf seiner Unterseite zumindest teilweise nicht klebend ausgeführt. Diese kann entweder dadurch erzielt werden, dass die Unterseite des Kennzeichnungsetiketts nur im Bereich des ersten und dritten Etikettenabschnitts mit Klebstoff beschichtet wurde. Alternativ kann das Kennzeichnungsetikett auf der Unterseite vollständig mit Klebstoff beschichtet werden und anschließend wird in dem Bereich des zweiten Etikettenabschnitts 2 die Klebekraft des Klebstoffes durch einen Klebstoffkiller neutralisiert. Ein Klebstoffkiller ist eine Substanz, die die Klebekraft des aufgetragenen Klebstoffs neutralisiert oder zumindest abschwächt.

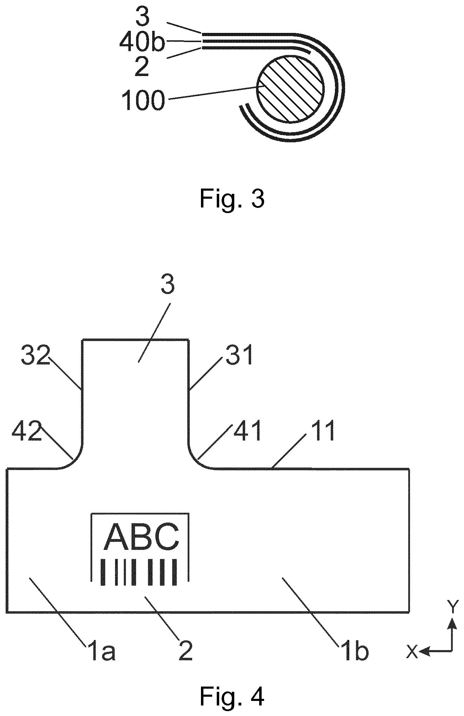

Wie in Figur 3 dargestellt ist, führen die nicht klebende Unterseite des zweiten Etikettenabschnitts 2 dazu, dass nach dem Aufbringen des Kennzeichnungsetiketts auf der zylinderförmigen Mantelfläche sich der zweite Kennzeichnungsabschnitt 2 von der Mantelfläche des zylinderförmigen Gefäßes 100 abhebt und annähernd gerade verläuft oder nur mit minimaler Krümmung absteht. Ein so abstehender Abschnitt des Kennzeichnungsetikett kann dann sowohl von einem Menschen als auch von einer geeigneten automatischen Leseeinrichtung sehr einfach erfasst werden, ohne dass das gekennzeichnete Produkt hin und her bewegt werden muss.

Als Kennzeichnung 20 auf der Oberseite des zweiten Etikettenabschnitts 2 können dabei beispielsweise in normaler Schrift aufgedruckte Schriftzeichen wie Buchstaben und Ziffern verwendet werden. Darüber hinaus eignet sich für eine automatische Erfassung auch ganz besonders aufgedruckte ein- oder zweidimensionale Codesequenzen, wie beispielsweise ein Barcode oder ein Datamatrixcode.

Dabei kann einerseits für ganze Chargen, die eine gemeinsame Kennzeichnung erhalten sollen, eine entsprechende Bedruckung bereits während des Herstellungsprozesses der Kennzeichnungsetiketten erfolgen. Andererseits können die Kennzeichnungsetiketten in einem nachgeordneten Prozess jeweils mit einer individuellen Kennzeichnung versehen werden, so dass jedes Kennzeichnungsetikett für sich ein Unikat darstellt. Für diese individuelle Kennzeichnung der einzelnen Etiketten eignet sich, wie bereits angeführt, ganz besonders eine Beschriftung mittels Tintenstrahldrucker oder Themotransferdrucker. Auch eine manuelle Beschriftung durch einen Menschen mittels eines Stiftes ist ebenso denkbar.

Da gerade individuellen Beschriftungen in der Regel in einem Verfahren erfolgen, bei dem die Beschriftung sehr leicht beschädigt oder auch manipuliert werden könnte, ist es besonders vorteilhaft, dass die Kennzeichnung beim Aufbringen des Etiketts auf dem zylinderförmigen Produkt gleichzeitig mit dem dritten Etikettenabschnitt 3 übersiegelt und somit geschützt wird.

Als Basismaterial für das oben beschriebene Kennzeichnungsetikett eignet sich besonders eine transparente Kunststofffolie, beispielsweise aus Polyester. Aber auch andere geeignete transparente Kunststofffolien sind ebenso möglich. Vorzugsweise kommen Folien mit einer Dicke von etwa 38 Mikrometer zum Einsatz.

Kennzeichnungsetiketten aus diesen Materialien besitzen in der Regel eine relativ gute Einreißfestigkeit, d.h. ein unbeschädigtes Etikett besitzt eine gute Resistenz gegen einen beginnenden Riss. Hat das Etikett jedoch bereits einmal eine Beschädigung erhalten und ist an einer Stelle eingerissen, so kann sich dieser Riss sehr rasch durch den gesamten Etikettenaufbau ausbreiten.

Bei einem nicht dargestellten Etikettenaufbau mit einem abstehenden Element besteht die Gefahr, dass das abstehende Kennzeichnungselement abgerissen werden könnte. Besonders gefährdet ist bei dem oben beschriebenen Aufbau dabei die nicht dargestellte Ecke, in der die Außenkante 11 des ersten Etikettenabschnitts 1 und die Außenkante 31 des dritten Etikettenabschnitts 3 zusammenstoßen. Der Scheitelpunkt des rechten Winkels zwischen den beiden Außenkanten 11 und 31 kann beim Stanzen während des Produktionsprozesses sehr leicht eine geringfügige Beschädigung erhalten, was einem minimalen Riss gleichkommt. Somit kann von dieser Stelle ausgehend der abstehende Etikettenteil sehr leicht abreißen.

Um dieser initialen Rissbildung entgegenzuwirken, wird bei dem erfindungsgemäßen Kennzeichnungsetikett der Übergang 41 zwischen den beiden Kanten 11 und 31 als Kreissegment ausgeführt. Vorzugsweise hat das Kreissegment einen Radius von 1 Millimeter oder mehr. Somit entsteht im Übergang zwischen den Kanten 11 und 31 kein ausgeprägter Winkel mit einem spitzen Scheitelpunkt, sondern ein sanfter Übergang. Dies führt dazu, dass der anstehende Kennzeichnungsabschnitt erheblich resistenter gegen Einreißen wird. Auf diese Weise kann die Gefahr eines Abreißens des Kennzeichnungsabschnitts ganz erheblich reduziert werden.

Figur 4 zeigt eine alternative Ausführungsform des erfindungsgemäßen Etiketts. Hierbei sind der zweite und dritte Etikettenabschnitt 2 und 3 nicht am Rand des Kennzeichnungsetiketts angeordnet, sondern liegen in einem mittleren Bereich des Kennzeichnungsetiketts. Der erste Etikettenabschnitt 1a und 1b ist in diesem Fall zweigeteilt und befindet sich jeweils links und rechts von den Etikettenabschnitten 2 und 3.

Mit anderen Worten, ist in X-Richtung der verlaufenden Achse zunächst der Teil 1a des ersten Etikettenabschnitts angeordnet, darauf schließt sich zunächst der zweite Etikettenabschnitt 2 und schließlich der Teil 1b des ersten Etikettenabschnitts an. Der Abschnitt 2 ist somit in X-Richtung vom den Abschnitten 1a und 1b umgeben. Wie in Figur 1 ist auch in diesem Fall der Verlauf der Y-Richtung orthogonal zur X-Richtung. Entlang der Y-Richtung schließt sich an den zweiten Etikettenabschnitt 2 auch hier der dritte Abschnitt 3 an.

Wird ein solches Kennzeichnungsetikett auf ein zylinderförmiges Gefäß aufgebracht, so befindet sich die abstehende Fahne mit der Kennzeichnung etwa in der Mitte des Gefäßes, was bei bestimmten Anwendungsfällen von Vorteil ist.

Zur Steigerung des Schutzes gegen ein unbeabsichtigtes Abreißen der Kennzeichnungsfahne sind in diesem Fall die beiden Übergänge 41 und 42 der beiden Kanten 31 und 32 des dritten Etikettenabschnitts zu den beiden Teilen des ersten Etikettenabschnitts 1a und 1b als Kreissegmente ausgeführt.

Zusammenfassend erhält man bei dem erfindungsgemäßen Etikettenaufbau ein Kennzeichnungsetikett für Spritzen und andere zylinderförmigen Produkte mit engen Radien, bei dem ein Kennzeichnungsabschnitt relativ gerade absteht, so dass er manuell und automatisch relativ einfach gelesen werden kann. Die Kennzeichnung kann dabei mit einem handelsüblichen Verfahren auch unabhängig von der Produktion des eigentlichen Kennzeichnungsetiketts erfolgen. Nach Aufbringen des Etiketts auf dem zylinderförmigen Körper wird die Beschriftung durch Versiegelung mit einem transparenten Laminat vor Verunreinigungen, Beschädigungen oder Manipulationen geschützt. Durch das Vermeiden eines Winkels mit einem spitzen Scheitelpunkt im Übergangsbereich 41 zwischen der Außenkante 11 ersten Etikettenabschnitts und Außenkante 31 des dritten Etikettenabschnitts wird die Einreißfestigkeit des Kennzeichnungsetikett signifikant erhöht.

## Patentansprüche

1. Kennzeichnungsetikett für ein zylinderförmiges Gefäß, umfassend
- einen ersten Etikettenabschnitt (1) mit einer mit Klebstoff (40a) beschichteten Unterseite (1a),
- einen opaken zweiten Etikettenabschnitt (2) mit einer nicht klebenden Unterseite (2a),
- einen transparenten dritten Etikettenabschnitt (3) mit einer mit Klebstoff (40b) beschichteten Unterseite (3a) und
- eine oder mehrere Stanzlinien (21), durch die der zweite Etikettenabschnitt (2) von dem ersten Etikettenabschnitt (1) und von dem dritten Etikettenabschnitt (3) getrennt ist,
**dadurch gekennzeichnet, dass**
- ein Übergang (41) zwischen einer ersten Außenkante (11) des ersten Etikettenabschnitts (1) und einer zweiten Außenkante (31) des dritten Etikettenabschnitts (3) einen abgerundeten Linienverlauf aufweist,
- dass der zweite Etikettenabschnitt (2) nur durch einen nicht eingestanzten Verbindungsbereich (22) mit dem ersten Etikettenabschnitt (1) verbunden ist,
- dass in einem auf einem zylinderförmigen Gefäß applizierten Zustand des Kennzeichnungsetiketts der dritte Etikettenabschnitt (3) über dem zweiten Etikettenabschnitt (2) zu liegen kommt und
- dass dadurch mittels des zweiten und dritten Etikettenabschnitts (2, 3) eine Fahne ausbildbar ist, die in dem applizierten Zustand des Kennzeichnungsetiketts von dem zylinderförmigen Gefäß absteht.

2. Kennzeichnungsetikett nach Anspruch 1, wobei die Unterseite (2a) des zweiten Etikettenabschnitts (2) mit Klebstoff beschichtet ist und der Klebstoff neutralisiert ist.

3. Kennzeichnungsetikett nach Anspruch 1 oder 2, wobei die Oberseite (2b) des zweiten Etikettenabschnitts (2) mit einer opaken Farbe (50) beschichtet ist.

4. Kennzeichnungsetikett nach einem der Ansprüche 1 bis 3, wobei der erste Etikettenabschnitt (1), der zweite Etikettenabschnitt (2) und der dritte Etikettenabschnitt aus einer gemeinsamen Folie, insbesondere einer gemeinsamen Folie aus einem transparenten Kunststoff hergestellt sind.

5. Kennzeichnungsetikett nach einem der Ansprüche 1 bis 4, wobei der abgerundete Linienverlauf des Übergangs (41) zwischen der ersten Außenkante (11) und der zweiten Außenkante (31) als Kreissegment, insbesondere mit einem Radius von mindestens 1 mm ausgebildet ist.

6. Kennzeichnungsetikett nach einem der Ansprüche 1 bis 5, wobei der zweite Etikettenabschnitt (2) einen Bereich mit aufgedruckten Informationen (20), insbesondere in Form eines maschinenlesbaren Codes umfasst.

7. Kennzeichnungsetikett nach einem der Ansprüche 1 bis 6, wobei das Kennzeichnungsetikett eine gemeinsame Kante des ersten Etikettenabschnitts (1) und des zweiten Etikettenabschnitts (2) aufweist, die zum Ankleben an eine zylindrische Umfangsfläche eines zylinderförmigen Gefäßes bestimmt ist.

8. Kennzeichnungsetikett nach Anspruch 7, wobei sich der Verbindungsbereich (22) von der gemeinsamen Kante des ersten und des zweiten Etikettenabschnitts (1, 2) bis zur Stanzlinie (21) erstreckt.

9. Kennzeichnungsetikett nach einem der Ansprüche 1 bis 8, wobei ein durch die Stanzlinie oder Stanzlinien (21) umgebener Teilbereich des zweiten Etikettenabschnitts (2) dem Flächenbereich der ausbildbaren Fahne entspricht.

10. Kennzeichnungsetikett nach einem der Ansprüche 1 bis 9, wobei die Stanzlinie (21) mindestens einen Stanzlinienabschnitt, der entlang einer Richtung (Y) verläuft, und einen Stanzlinienabschnitt, der entlang einer dazu orthogonalen Richtung (X) verläuft, umfasst.

11. Kennzeichnungsetikett nach einem der Ansprüche 1 bis 10, wobei die Stanzlinie (21) zwei Stanzlinienabschnitte, die entlang einer Richtung (Y) verlaufen, und dazwischen einen Stanzlinienabschnitt, der entlang einer dazu orthogonalen Richtung (X) verläuft, umfasst und wobei der zweite Etikettenabschnitt (2) in orthogonaler Richtung (X) auf beiden Seiten nur durch einen jeweiligen Verbindungsbereich (22) mit dem jeweiligen ersten Etikettenabschnitt (1a; 1b) verbunden ist.

12. Zylinderförmiges Gefäß (100) mit einem applizierten Kennzeichnungsetikett nach einem der Ansprüche 1 bis 11, wobei das zylinderförmige Gefäß eine Zylinderachse aufweist und das Kennzeichnungsetikett so auf dem zylinderförmigen Gefäß (100) aufgebracht ist, dass die erste Außenkante (11) des ersten Etikettenabschnitts (1) parallel zur Zylinderachse verläuft.

13. Zylinderförmiges Gefäß (100) nach Anspruch 12, wobei das Kennzeichnungsetikett so auf dem zylinderförmigen Gefäß (100) aufgebracht ist, dass der dritte Etikettenabschnitt (3) den zweiten Etikettenabschnitt (2) überdeckt.

14. Zylinderförmiges Gefäß (100) nach Anspruch 12 oder 13, wobei der zweite Etikettenabschnitt (2) und der dritte Etikettenabschnitt (3) eine Fahne bilden, die von dem zylinderförmigen Gefäß (100) absteht.

15. Zylinderförmiges Gefäß (100) nach einem der Ansprüche 12 bis 14, wobei das zylinderförmige Gefäß (100) ein Spritzenkörper einer Injektionsspritze ist.

## Claims

1. An identification label for a cylindrical container, comprising
- a first label portion (1) having an underside (1a) which is coated with an adhesive (40a),
- an opaque second label portion (2) having a non-adhesive underside (2a),
- a transparent third label portion (3) having an underside (3a) which is coated with an adhesive (40b), and
- one or more stamping lines (21) by means of which the second label portion (2) is separated from the first label portion (1) and the third label portion (3),
**characterized in that**
- a transition (41) between a first outer edge (11) of the first label portion (1) and a second outer edge (31) of the third label portion (3) has a rounded line progression,
- that the second label portion (2) is connected to the first label portion (1) only by a non-stamped connection area (22),
- that the third label portion (3) comes to lie over the second label portion (2) in a state when the identification label is applied to a cylindrical container and
- that the second and third label portion (2, 3) are thus capable of forming a lug which in the applied state of the identification label protrudes from the cylindrical container.

2. The identification label according to claim 1, wherein the underside (2a) of the second label portion (2) is coated with an adhesive and the adhesive is neutralized.

3. The identification label according to claim 1 or 2, wherein the upper side (2b) of the second label portion (2) is coated with an opaque color (50).

4. The identification label according to any of the claims 1 to 3, wherein the first label portion (1), the second label portion (2) and the third label portion are made from a common foil, in particular a common foil of transparent plastic.

5. The identification label according to any of the claims 1 to 4, wherein the rounded line progression of the transition (41) between the first outer edge (11) and the second outer edge (31) is formed as a circular segment, in particular with a radius of at least 1 mm.

6. The identification label according to any of the claims 1 to 5, wherein the second label portion (2) comprises an area with printed information (20), in particular in the form of a machine-readable code.

7. The identification label according to any of the claims 1 to 6, wherein the identification label comprises a common edge of the first label portion (1) and of the second label portion (2), which is intended for being adhered to a cylindrical circumferential surface of a cylindrical container.

8. The identification label according to claim 7, wherein the connection area (22) extends from the common edge of the first and the second label portion (1, 2) to the stamping line (21).

9. The identification label according to any of the claims 1 to 8, wherein a partial area of the second label portion (2) surrounded by the stamping line(s) (21) corresponds to the surface region of the formable lug.

10. The identification label according to any of the claims 1 to 9, wherein the stamping line (21) comprises at least one stamping line portion extending along a direction (Y), and a stamping line portion extending along a direction (X) orthogonal thereto.

11. The identification label according to any of the claims 1 to 10, wherein the stamping line (21) comprises two stamping line portions extending along a direction (Y), and therebetween a stamping line portion extending along a direction (X) orthogonal thereto, and wherein the second label portion (2), in the orthogonal direction (X), is connected on both sides to the respective first label portion (1a; 1b) only by a respective connection area (22).

12. A cylindrical container (100) having an applied identification label according to any of the claims 1 to 11, wherein the cylindrical container has a cylinder axis and the identification label is applied to the cylindrical container (100) in such a manner that the first outer edge (11) of the first label portion (1) extends parallel to the cylinder axis.

13. The cylindrical container (100) according to claim 12, wherein the identification label is applied to the cylindrical container (100) in such a manner that the third label portion (3) covers the second label portion (2).

14. The cylindrical container (100) according to claim 12 or 13, wherein the second label portion (2) and the third label portion (3) form a lug which protrudes from the cylindrical container (100).

15. The cylindrical container (100) according to any of the claims 12 to 14, wherein the cylindrical container (100) is a syringe body of an injection syringe.

## Revendications

1. Étiquette de marquage pour un récipient cylindrique, comprenant
- une première section d'étiquette (1) présentant une face inférieure (1a) revêtue de colle (40a),
- une deuxième section d'étiquette (2) opaque présentant une face inférieure (2a) non adhésive,
- une troisième section d'étiquette (3) transparente présentant une face inférieure (3a) revêtue de colle (40b) et
- une ou plusieurs lignes de découpe (21) par lesquelles la deuxième section d'étiquette (2) est séparée de la première section d'étiquette (1) et de la troisième section d'étiquette (3),
**caractérisée en ce que**
- une transition (41) entre un premier bord extérieur (11) de la première section d'étiquette (1) et un deuxième bord extérieur (31) de la troisième section d'étiquette (3) présente un tracé linéaire arrondi,
- la deuxième section d'étiquette (2) n'est reliée à la première section d'étiquette (1) que par une zone de liaison (22) non découpée,
- dans un état appliqué sur un récipient cylindrique de l'étiquette de marquage, la troisième section d'étiquette (3) vient reposer par-dessus la deuxième étiquette (2) et
- de la sorte, moyennant la deuxième et la troisième section d'étiquette (2, 3), une languette qui fait saillie par rapport au récipient cylindrique dans l'état appliqué de l'étiquette de marquage peut être constituée.

2. Étiquette de marquage selon la revendication 1, sachant que la face inférieure (2a) de la deuxième section d'étiquette (2) est revêtue de colle et la colle est neutralisée.

3. Étiquette de marquage selon la revendication 1 ou 2, sachant que la face supérieure (2b) de la deuxième section d'étiquette (2) est revêtue d'une couleur (50) opaque.

4. Étiquette de marquage selon l'une des revendications 1 à 3, sachant que la première section d'étiquette (1), la deuxième section d'étiquette (2) et la troisième section d'étiquette sont fabriquées à partir d'une feuille commune, en particulier d'une feuille commune en matière synthétique transparente.

5. Étiquette de marquage selon l'une des revendications 1 à 4, sachant que le tracé linéaire arrondi de la transition (41) entre le premier bord extérieur (11) et le deuxième bord extérieur (31) est constitué comme segment de cercle, en particulier d'un rayon d'au moins 1 mm.

6. Étiquette de marquage selon l'une des revendications 1 à 5, sachant que la deuxième section d'étiquette (2) comprend une zone avec des informations (20) imprimées, en particulier sous forme d'un code lisible par machine.

7. Étiquette de marquage selon l'une des revendications 1 à 6, sachant que l'étiquette de marquage présente un bord commun de la première section d'étiquette (1) et de la deuxième section d'étiquette (2) qui est destiné à coller à une face périphérique cylindrique d'un récipient cylindrique.

8. Étiquette de marquage selon la revendication 7, sachant que la zone de liaison (22) s'étend depuis le bord commun de la première et de la deuxième section d'étiquette (1, 2) jusqu'à la ligne de découpe (21).

9. Étiquette de marquage selon l'une des revendications 1 à 8, sachant qu'une zone partielle de la deuxième section d'étiquette (2) entourée par la ligne de découpe ou des lignes de découpe (21) correspond à la zone surfacique de la languette pouvant être constituée.

10. Étiquette de marquage selon l'une des revendications 1 à 9, sachant que la ligne de découpe (21) comprend au moins une section de ligne de découpe qui passe le long d'une direction (Y), et une section de ligne de découpe qui passe le long d'une direction (X) perpendiculaire à celle-ci.

11. Étiquette de marquage selon l'une des revendications 1 à 10, sachant que la ligne de découpe (21) comprend deux sections de ligne de découpe qui passent le long d'une direction (Y), et entre elles une section de ligne de découpe qui passe le long d'une direction (X) perpendiculaire à celle-ci, et sachant que la deuxième section d'étiquette (2) n'est reliée des deux côtés à la première section d'étiquette (la ; 1b) respective en direction (X) perpendiculaire que par une zone de liaison (22) respective.

12. Récipient cylindrique (100) avec une étiquette de marquage appliquée selon l'une des revendications 1 à 11, sachant que le récipient cylindrique présente un axe de cylindre et l'étiquette de marquage est apposée sur le récipient cylindrique (100) de telle façon que le premier bord extérieur (11) de la première section d'étiquette (1) soit parallèle à l'axe de cylindre.

13. Récipient cylindrique (100) selon la revendication 12, sachant que l'étiquette de marquage est apposée sur le récipient cylindrique (100) de telle façon que la troisième section d'étiquette (3) recouvre la deuxième section d'étiquette (2).

14. Récipient cylindrique (100) selon la revendication 12 ou 13, sachant que la deuxième section d'étiquette (2) et la troisième section d'étiquette (3) forment une languette qui fait saillie par rapport au récipient cylindrique (100).

15. Récipient cylindrique (100) selon l'une des revendications 12 à 14, sachant que le récipient cylindrique (100) est un corps de seringue d'une seringue d'injection.
